# EUROPÄISCHE PATENTSCHRIFT

(11) **EP 2 480 525 B1**
(45) Veröffentlichungstag und Bekanntmachung des Hinweises auf die Patenterteilung: **17.04.2013**
(21) Anmeldenummer: 10752347.4
(22) Anmeldetag: 09.09.2010
(51) Int. Cl.: C07C 263/06, C07C 263/04

(54) **VERFAHREN ZUR HERSTELLUNG VON ISOCYANATEN**
METHOD FOR PRODUCING ISOCYANATES
PROCÉDÉ DE PRODUCTION D'ISOCYANATES

(30) Priorität: 22.09.2009 EP 09170992
(43) Veröffentlichungstag der Anmeldung: 01.08.2012
(73) Patentinhaber: BASF SE, 67056 Ludwigshafen (DE)
(72) Erfinder: MATTKE, Torsten, 67251 Freinsheim (DE); KLÖTZER, Matthias, 01945 Kroppen (DE); STROEFER, Eckhard, 68163 Mannheim (DE); GEIßLER, Bernhard, 67281 Kirchheim (DE); ZEHNER, Peter, 67273 Weisenheim am Berg (DE)
(86) Internationale Anmeldenummer: PCT/EP2010/063225
(87) Internationale Veröffentlichungsnummer: WO 2011/036062

(56) Entgegenhaltungen:
- EP-A1- 0 657 420
- EP-A1- 1 593 669
- WO-A1-2007/031444

## Beschreibung

Gegenstand der Erfindung ist ein mehrstufiges Verfahren zur kontinuierlichen Herstellung von organischen, destillierbaren Polyisocyanaten, vorzugsweise von Diisocyanaten, besonders bevorzugt von aliphatischen oder cycloaliphatischen Diisocyanaten, durch Umsetzung der entsprechenden organischen Polyamine mit Kohlensäurederivaten und Alkoholen in niedermolekulare monomere Polyurethane und deren thermische Spaltung, bei dem an bestimmten Reaktionsstufen die hergestellten Polyisocyanate und unverwertbaren Rückstände abgetrennt und wiederverwendbare Neben- und Zwischenprodukte in Vorstufen zurückgeführt werden.

Die technischen Verfahren zur Herstellung von organischen Polyisocyanaten, wie z.B. von aromatischen, aliphatischen oder cycloaliphatischen Polyisocyanaten, beruhen auf der Phosgenierung der entsprechenden organischen Polyamine zu Polycarbamidsäurechloriden und deren thermische Spaltung zu den Polyisocyanaten und Chlorwasserstoff. Abgesehen von den schwerwiegenden Umweltschutz-, Entsorgungs- und Sicherheitsproblemen, die der Einsatz von Phosgen mit sich bringt, sind diese Verfahren mit weiteren entscheidenden Nachteilen behaftet. So gelingt die Herstellung von aliphatischen oder cycloaliphatischen Polyisocyanaten aufgrund der stärkeren Basizität der Ausgangspolyamine nur mit recht mäßigen Raum-Zeit-Ausbeuten. Nachteilig ist ferner die Bildung von unerwünschten Nebenprodukten, die, bereits in Spuren vorliegend, zu starken Verfärbungen der Polyisocyanate führen können. Bei der Hexamethylendiisocyanat-1,6 (HDI)-Herstellung entstehen z.B. mehrere Nebenprodukte, von denen das wichtigste, 6-Chlorhexylisocyanat, zudem den Nachteil besitzt, daß es nur mit erheblichem destillativen Aufwand vom HDI abgetrennt werden kann.

Problematisch bei dieser Verfahrensweise sind insbesondere der hohe Umsatz von Chlor über Phosgen und Carbamidsäurechlorid in Chlorwasserstoff, die Toxizität des Phosgens sowie die Korrosivität des Reaktionsgemisches, die Labilität der in der Regel eingesetzten Lösungsmittel und die Bildung halogenhaltiger Rückstände.

Obwohl die thermische Spaltung von (cyclo)aliphatischen und insbesondere aromatischen Mono- und Diurethanen in die entsprechenden Isocyanate und Alkohol seit langem bekannt ist und sowohl in der Gasphase bei hohen Temperaturen als auch in der flüssigen Phase bei vergleichsweise niedrigen Temperaturen ausgeführt werden kann, sind es besonders die unerwünschten Nebenreaktionen und vor allem die Tendenz der Reaktionsmischungen zur Ausbildung von Belegungen, Verharzungen und Verstopfungen in Reaktoren und Aufarbeitungseinrichtungen, die die Wirtschaftlichkeit der Prozesse nachhaltig beeinträchtigen.

In den vergangenen Jahrzehnten hat es deshalb viele Anstrengungen gegeben, diese Nachteile des Verfahrens durch ein einfacheres und verbessertes Verfahren zu beseitigen. So wurden zur Herstellung von aliphatischen und/oder cycloaliphatischen Di- und/oder Polyurethanen gemäß EP 18588 A1 oder auch wie in EP 28338 A2 primäre aliphatische und/oder cycloaliphatische Di- und/oder Polyamine mit O-Alkylcarbamidsäureestern in Gegenwart von Alkoholen bei Temperaturen von 160 bis 300 °C mit und ohne Katalysator umgesetzt. Die entstandenen Di- und/oder Polyurethane können in die entsprechenden Isocyanate überführt werden. Der bei der Umsetzung der Amine entstehende Ammoniak kann dabei abgetrennt werden.

Weitere Veröffentlichungen befassen sich mit der teilweisen Substitution von Harnstoff und/oder Diaminen durch carbonylgruppenhaltige Verbindungen (z.B. EP 27952 oder EP 126299). Das phosgenfreie Verfahren wird ausführlich beispielsweise in EP 566925 A2 beschrieben.

Nachteilig am letzteren Verfahren ist die relativ lange Reaktionszeit, die mit bis zu 50 Stunden angegeben wird.

Aus WO 2007/31444 ist ein Verfahren zur Herstellung von Isocyanaten durch Spaltung der entsprechenden Urethane bekannt, in dem das korrespondierende Amin mit Harnstoff und mindestens einem Alkohol gemeinsam in einer Mischeinrichtung vermischt und miteinander umgesetzt wird.

In der Mischeinrichtung findet ein Umsatz bis zu 10 % statt, der restliche Umsatz erfolgt in einem angeschlossenen Reaktorsystem.

Obwohl diese Reaktionsführung bereits stark optimiert ist, ist eine weitere Verringerung der in der Reaktion gebildeten Nebenprodukte gewünscht.

Die Aufgabe der vorliegenden Erfindung bestand darin, destillierbare organische Polyisocyanate, insbesondere aliphatische und cycloaliphatische Diisocyanate, mit hoher Selektivität in verbesserten Raum-Zeit-Ausbeuten kostengünstig auf einfache Weise ohne die Verwendung von kostspieligen und/oder sicherheitsgefährdenden Ausgangs- oder Hilfsstoffen herzustellen. Insbesondere sollen weniger Nebenprodukte als nach den Verfahren des Standes der Technik gebildet werden.

Diese Aufgabe konnte gelöst werden durch ein Verfahren zur Herstellung von Isocyanaten durch Umsetzung mindestens eines Amins mit Harnstoff und mindestens einem Alkohol zum korrespondierenden Urethan in mindestens einer Mischeinrichtung mit mindestens einem angeschlossenem Verweilzeitreaktor und anschließender Spaltung der so erhaltenen Urethane in die korrespondierenden Isocyanate, in dem man zumindest einen Teil des Amins und zumindest einen Teil des mindestens einen Alkohols als Gemisch in der mindestens einen Mischeinrichtung mit Harnstoff sowie dem gegebenenfalls verbliebenen restlichen Amin sowie dem gegebenenfalls verbliebenen restlichen Alkohol vermischt und reagieren läßt.

Gegenstand der Erfindung ist ferner ein mehrstufiges Verfahren zur kontinuierlichen Herstellung von organischen Isocyanaten durch Umsetzung der entsprechenden organischen Amine mit Harnstoff und mindestens einem Alkohol in die entsprechenden Urethane in mindestens zwei Mischeinrichtungen mit nachgeschaltetem Reaktor und deren thermische Spaltung, das folgende Stufen umfaßt und in dem man
0) zumindest einen Teil mindestens eines, bevorzugt genau eines organischen Amins und zumindest einen Teil mindestens eines, bevorzugt genau eines Alkohols optional in Gegenwart oder vorzugsweise in Abwesenheit mindestens eines Katalysators in mindestens einer ersten Mischeinrichtung zu einer Vormischung vermischt,
a) diese Vormischung sowie optional das restliche organische Amin sowie optional den restlichen weiteren Alkohol mit Harnstoff in Gegenwart oder vorzugsweise in Abwesenheit mindestens eines Katalysators in mindestens einer zweiten Mischeinrichtung teilweise miteinander reagieren läßt,
b) das aus a) erhaltene Gemisch in mindestens einem sich anschließenden Verweilzeitreaktor oder mehreren Verweilzeitreaktoren, die von ihrer Verweilzeitverteilung einem Rohrreaktor ähneln, im wesentlichen vollständig umsetzt
c) den dabei entstehenden Ammoniak abtrennt,
d) aus dem Austrag aus c) überschüssigen Alkohol und weitere leichtsiedende Nebenkomponenten abtrennt,
e) das vom Alkohol und leichtsiedenden Komponenten befreite Urethan aus (d) zumindest teilweise einer Destillation zuführt,
f) die Urethane im Destillat aus (e) und den gegebenenfalls nicht der Destillation (e) zugeführten Anteil aus (d) in einer kontinuierlichen Spalteinrichtung in das entsprechende Isocyanat und Alkohol spaltet,
g) das aus (f) erhaltene Rohisocyanat in mindestens einer Destillation reinigt und anfallende Destillationsrückstände erneut der Spaltung (f) zuführt und/oder mit Alkohol zu Urethanen umwandelt und der Reaktionseinheit (b) zuführt und
h) den Reaktionsaustrag aus (f), der einen hohen Anteil an Urethanen und verwertbaren Verbindungen enthält, erneut in Urethane durch Umsatz mit Alkoholen überführt wird.

Das erfindungsgemäße Verfahren weist zum Erreichen eines vorgegebenen Umsatzes kürzere Verweilzeiten und somit eine bessere Raum-Zeit-Ausbeute auf, als im Stand der Technik bekannte Verfahren, insbesondere das aus der EP 566 925 bekannte Verfahren. Gegenüber der WO 2007/31444 erbringt das erfindungsgemäße Verfahren ein Produktgemisch mit einem geringeren Anteil an Nebenprodukten.

Rein formal betrachtet kann das erfindungsgemäße Verfahren schematisch durch folgende Gleichung bilanziert werden:

R-(NH₂)ₙ + n H₂N(CO)NH₂ + n R'OH → R(NCO)ₙ + n R'OH + 2n NH₃

Zur Herstellung der erfindungsgemäß als Zwischenprodukte verwendbaren monomeren Polyurethane eignen sich Amine der Formel R(NH₂)ₙ, in der R einen mehrwertigen, vorzugsweise zweiwertigen organischen Rest, wie z.B. einen gegebenenfalls substituierten, beispielsweise mit einer Alkylgruppe substituierten aromatischen oder vorzugsweise einen linearen oder verzweigtkettigen, aliphatischen oder gegebenenfalls substituierten cycloaliphatischen Rest bedeutet.

Als geeigente aromatische Polyamine beispielhaft genannt seien 2,4- und 2,6-Toluylen-diamin, 4,4'-, 2,4'- und 2,2'-Diamino-diphenylmethane und die entsprechenden lsomerengemische.

Als aliphatische oder cycloaliphatische Polyamine kommen beispielsweise in Betracht: Butandiamin-1,4, 2-Ethylbutandiamin-1,4, Octandiamin-1,8, Decandiamin-1,10, Dodecandiamin-1,12, Cyclohexandiamin-1,4, 2-Methyl-, 4-Methyl-cyclohexandiamin-1,3, und 1,3- und 1,4-Diaminomethylcyclohexan3 (bzw. 4), 8 (bzw. 9)-Bis(aminomethyl)-tricyclo[5.2.1.0^{2.6}]decan-Isomerengemische. Vorzugsweise Verwendung finden 2-Methylpentandiamin-1,5, 2,2,4- bzw. 2,4,4-Trimethylhexandiamin-1,6 und insbesondere Hexandiamin-1,6 und 3-Aminomethyl-3,5,5-trimethylcyclohexylamin.

Der Alkohol kann beispielsweise 2-18, besonders bevorzugt 2-7 Kohlenstoffatome aufweisen. Der Alkohol kann unverzweigt, verzweigt oder cyclisch sein.

In einer Ausführungsform der Erfindung kann die Alkylkette des entsprechenden Alkohols mit Heteroatomen modifiziert sein. Bei den Heteroatomen kann es sich um Halogenatome handeln, vorzugsweise um Fluoratome und/oder Chloratome, besonders bevorzugt um Fluoratome. In einer anderen Ausführungsform handelt es sich bei den Heteroatomen um Sauerstoffatome. Diese liegen bevorzugt als Ethergruppen vor. Bevorzugte Alkohole sind alle aliphatischen Alkohole. Vorzugsweise wird man jedoch solche auswählen, deren Siedepunkte genügend weit vom Siedepunkt des durch die thermische Spaltung erhaltenen Polyisocyanates, vorzugsweise Diisocyanates, entfernt liegen, so daß eine möglichst quantitative Trennung der Spaltprodukte Polyisocyanat, vorzugsweise Diisocyanat und Alkohol möglich ist.

Aus diesen Gründen finden daher vorzugsweise Alkohole, wie z.B. Methanol, Ethanol, n-Propanol, n-Butanol, iso-Butanol, n-Pentanol, iso-Pentanol, n-Hexanol, Isohexanole, 2-Ethylhexanol, Decanol oder Gemische der genannten Alkohole, insbesondere aber n-Butanol und/oder iso-Butanol Verwendung.

Die einzelnen Stufen des Verfahrens werden im Folgenden beschrieben:
0) Vormischung von Amin und Alkohol

In der Stufe (0) werden zumindest ein Teil des gesamten in die Reaktion eingesetzen Amins sowie zumindest ein Teil des gesamten in die Reaktion eingesetzen Alkohols in einer ersten Mischeinrichtung miteinander vermischt.

Bevorzugt werden 10 bis 100% des gesamten in die Reaktion eingesetzen Amins in die Stufe (0) geführt, besonders bevorzugt 25 bis 100%, ganz besonders bevorzugt 50 bis 100%, insbesondere 75 bis 100% und speziell 100%.

Bevorzugt werden 10 bis 100% des gesamten in die Reaktion eingesetzen Alkohols in die Stufe (0) geführt, besonders bevorzugt 25 bis 100%, ganz besonders bevorzugt 50 bis 100%, insbesondere 75 bis 100% und speziell 100%.

Für die Bestimmung der gesamten in die Reaktion eingesetzen Amins bzw. Alkohols wird die in Stufe (a) angestrebte Stöchiometrie zugrundegelegt (siehe unter Stufe a).

Sollte ein Katalysator in die Reaktion eingesetzt werden, so kann dieser bereits in die Stufe (0) eingemischt werden, beispielsweise zusammen mit dem zugeführten Aminstrom oder bevorzugt dem zugeführten Alkoholstrom, bevorzugt wird der gegebenenfalls eingesetzte Katalysator jedoch erst in Stufe (a) eingemischt.

Erfindungsgemäß wesentlich ist, daß zumindest ein Teil des Amins und zumindest ein Teil des mindestens einen Akohols in Form eines Gemisches, im Rahmen dieser Schrift als Vorgemisch oder Vormischung bezeichnet, in die Stufe (a) dosiert werden.

Auf welche Weise dieses Vorgemisch erzeugt wird, ist dabei nicht erfindungswesentlich.

Erfindungswesentlich ist, daß mindestens 95,5 % der Fluidelemente der Vormischung einen Mischungsbruch haben, der bezogen auf den Wert des theoretischen Endwert des Mischungsbruchs des erhaltenen Gemisches beim Erreichen des Zustandes perfekter Mischung weniger als 2 % von diesem Endwert des Mischungbruches abweichen (zum Konzept des Mischungsbruches siehe z.B. J.Warnatz, U.Maas, R.W. Dibble: Verbrennung, Springer Verlag, Berlin Heidelberg New York, 1997, 2. Auflage, S. 134.).

Bevorzugt werden zumindest ein Teil des Amins und zumindest ein Teil des mindestens einen Akohols jeweils in flüssiger Form miteinander vermischt.

Die Vermischung des mindestens einen, bevorzugt genau einen Amins mit dem mindestens einen, bevorzugt genau einen Alkohol erfolgt bei Temperaturen von Umgebungstemperatur bis 300 °C, bevorzugt 20 bis 250 °C und besonders bevorzugt bei 100 - 220 °C unter einem Druck von 0,1 bis 30 bar abs, vorzugsweise bei Umgebungsdruck bis 15 bar abs.

Bevorzugt wird die Temperatur in der Stufe (0) so gewählt, daß das Vorgemisch beim Übergang in Stufe (a) nicht weiter erhitzt werden braucht, sondern eine Temperatur aufweist, die ausreichend ist um nach Vermischung mit Harnstoff in Stufe (a) die erforderliche Reaktionstemperatur zu gewährleisten.

Sollte ein Teil des in die Stufe (0) geführten Alkohols Dialkylcarbonate und/oder Carbamidsäurealkylester enthalten, beispielsweise weil es sich um rückgeführten Alkohol aus der Stufe (d) handelt (siehe unten), so kann die Vormischung in Stufe (0) in Gegenwart von Dialkylcarbonaten, zweckmäßigerweise in einer Menge von 0,01 bis 30 Mol%, vorzugsweise 0,02 bis 10 Mol% und/oder Carbamidsäurealkylestern zweckmäßigerweise in einer Menge von 0,1 bis 5 Mol%, vorzugsweise von 0,2 bis 2 Mol%, bezogen auf das Amin erfolgen. Als Dialkylcarbonate und/oder Carbamidsäureester setzt man bevorzugt solche ein, deren Alkylreste dem Alkylrest des verwendeten Alkohols entsprechen.

Sollte Dialkylcarbonat und/oder Carbamidsäurealkylester in die Vormischung (0) geführt werden, so ist nicht auszuschließen, daß bereits während der Vormischung ein Teil der Reaktion mit dem Amin stattfindet.

Wie bereits dargelegt, kann die Umsetzung in der Vormischung (0) auch in Gegenwart von Katalysatoren erfolgen. Diese werden zweckmäßigerweise in Mengen von mindestens 1 Gew.ppm vorzugsweise mindestens 2 Gew.ppm insbesondere mindestens 5 Gew.ppm, bezogen auf das Gewicht der Reaktionsmischung, eingesetzt. In der Regel braucht die Menge des Katalysators nach oben nicht begrenzt zu werden, bevorzugt beträgt die Menge nicht mehr als 100 Gew.ppm, besonders bevorzugt nicht mehr als 50 Gew.ppm und ganz besonders bevorzugt nicht mehr als 20 Gew.ppm.

Die geeigneten Katalysatoren sind unten unter Stufe (a) näher beschrieben.

Die Vormischung der Eduktströme erfolgt im erfindungsgemäßen Verfahren in einer beliebigen Mischeinrichtung, die sich durch ausreichend Vermischung auszeichnet.

Als Mischeinrichtung bevorzugt wird ein Mischkreis, Mikromischer, ein Rührbehälter, Rührkesselkaskaden, eine Mischpumpe, Rotor-Stator-Systeme, statische Mischer oder eine Düsenmischeinrichtung, beispielsweise Querstrom-, Kammer-, Koaxialmischdüsen, Y- oder T-Mischer, oder eine Vortex-Impinging-Jet-Mischkonfiguration eingesetzt, bevorzugt sind gegebenenfalls kaskadierte Rührbehalter, statische Mischer und Y- oder T-Mischer.

Der Druck am Ausgang der Mischeinrichtung liegt in der Regel oberhalb des Drucks in Stufe (a), beispielsweise zwischen 5 und 100 bar, bevorzugt zwischen 10 und 80 bar, besonders bevorzugt zwischen 10 und 50 bar.

Der Austrag aus der Stufe (0) kann vor Einleiten in die Stufe (a) mit Hilfe eines Wärmetauschers auf die dort gewünschte Temperatur gebracht werden.

### a) Vermischung der Reaktionskomponenten

Zur Umsetzung in der Reaktionsstufe (a) werden die Vormischung aus Amin und Alkohol aus Stufe (0) mit Harnstoff sowie gegebenenfalls dem restlichen, nicht in die Stufe (0) eingesetzten Amin und/oder Alkohol in einem molaren Verhältnis von Amin, Harnstoff und Alkohol wie 1 : 2 bis 20 : 5 bis 40 bei Temperaturen von 50 - 300 °C und insbesondere bei 150 - 220 °C unter einem Druck von 0,1 bis 30 bar, vorzugsweise 5 - 20 bar vermischt und zur Reaktion gebracht. Bei diesen Reaktionsbedingungen ergeben sich für das erfindungsgemäße Verfahren mittlere Reaktionszeiten von Bruchteilen von Sekunden bis Minuten.

Die Umsetzung in der Stufe (a) verläuft im wesentlichen vom Harnstoff mit Amin zum korrespondierenden Harnstoff, also im Fall der Diamine im wesentlichen zu den Diharnstoffen. Es ist möglich, daß die so erhaltenen Harnstoffe bzw. Diharnstoffe bereits zu einem untergeordneten Teil zu den korrespondierenden Urethanen bzw. Diurethanen weiterreagieren. Letztere Reaktion findet im wesentlichen in Stufe b) statt.

Die Umsetzung in der Reaktionsstufe (a) kann in Gegenwart von Dialkylcarbonaten, zweckmäßigerweise in einer Menge von 0,01 bis 30 Mol%, vorzugsweise 0,02 bis 10 Mol% und/oder Carbamidsäurealkylestern zweckmäßigerweise in einer Menge von 0,1 bis 5 Mol%, vorzugsweise von 0,2 bis 2 Mol%, bezogen auf das Polyamin, vorzugsweise Diamin, durchgeführt werden. Insbesondere verwendet werden dabei Mischungen aus Dialkylcarbonaten und Carbamidsäurealkylestern in den genannten Mengenverhältnissen. Als Dialkylcarbonate und/oder Carbamidsäureester setzt man bevorzugt solche ein, deren Alkylreste dem Alkylrest des verwendeten Alkohols entsprechen.

Wie bereits dargelegt, kann die Umsetzung in der Reaktionsstufe (a) auch in Gegenwart von Katalysatoren erfolgen. Diese werden zweckmäßigerweise in Mengen von mindestens 1 Gew.ppm vorzugsweise mindestens 2 Gew.ppm insbesondere mindestens 5 Gew.ppm, bezogen auf das Gewicht der Reaktionsmischung, eingesetzt. In der Regel braucht die Menge des Katalysators nach oben nicht begrenzt zu werden, bevorzugt beträgt die Menge nicht mehr als 100 Gew.ppm, besonders bevorzugt nicht mehr als 50 Gew.ppm und ganz besonders bevorzugt nicht mehr als 20 Gew.ppm.

Als Katalysatoren eignen sich anorganische oder organische Verbindungen, die ein oder mehrere Kationen, vorzugsweise ein Kation von Metallen der Gruppe IA, IB, IIA, IIB, IIIB, IVA, IVB, VA, VB, VIB, VIIB, VIIIB des Periodensystems der Elemente enthalten, definiert gemäß Handbook of Chemistry and Physics 14th Edition, publiziert von Chemical Rubber Publishing Co., 23 Superior Ave. N.E., Cleveland, Ohio, enthalten. Beispielhaft genannt seien die Kationen folgender Metalle: Lithium, Natrium, Kalium, Magnesium, Calcium, Aluminium, Gallium, Zinn, Blei, Bismut, Antimon, Kupfer, Silber, Gold, Zink, Quecksilber, Cer, Titan, Vanadium, Chrom, Molybdän, Mangan, Eisen und Cobalt.

Der Katalysator kann weiterhin mindestens ein Anion enthalten, beispielsweise Halogenide, wie Chloride und Bromide, Sulfate, Phosphate, Nitrate, Borate, Alkoholate, Phenolate, Sulfonate, Oxide, Oxidhydrate, Hydroxide, Carboxylate, Chelate, Carbonate und Thio- oder Dithiocarbamate.

Die Katalysatoren können ohne erkennbare deutliche Nachteile auch in Form ihrer Hydrate oder Ammoniakate zum Einsatz kommen.

Als typische Katalysatoren seien beispielhaft folgende Verbindungen genannt: Lithiummethanolat, Lithiumethanolat, Lithiumpropanolat, Lithiumbutanolat, Natriummethanolat, Kalium-tert.-butanolat, Magnesiummethanolat, Calciummethanolat, Zinn-(II)-chlorid, Zinn-(IV)-chlorid, Bleiacetat, Bleiphosphat, Antimon-(III)-chlorid, Antimon-(V)-chlorid, Aluminiumacetylacetonat, Aluminium-iso-butylat, Aluminiumtrichlorid, Bismut-(III)-chlorid, Kupfer-(II)-acetat, Kupfer-(II)-sulfat, Kupfer-(II)-nitrat, Bis-(triphenylphosphinoxido)-kupfer-(II)-chlorid, Kupfermolybdat, Silberacetat, Goldacetat, Zinkoxid, Zinkchlorid, Zinkacetat, Zinkacetonylacetat, Zinkoctoat, Zinkoxalat, Zinkhexylat, Zinkbenzoat, Zinkundecylenat, Cer-(IV)-oxid, Uranylacetat, Titantetrabutanolat, Titantetrachlorid, Titantetraphenolat, Titannaphtenat, Vanadium-(III)-chlorid, Vanadiumacetylacetonat, Chrom-(III)-chlorid, Molybdän-(VI)-oxid, Molybdänacetylacetonat, Wolfram-(VI)-oxid, Mangan-(II)-chlorid, Mangan-(II)-acetat, Mangan-(III)-acetat, Eisen-(II)-acetat, Eisen-(III)-acetat, Eisenphosphat, Eisenoxalat, Eisen-(III)-chlorid, Eisen-(III)-bromid, Cobaltacetat, Cobaltchlorid, Cobaltsulfat, Cobaltnaphthenat, Nickelchlorid, Nickelacetat und Nickelnaphthenat sowie deren Gemische.

Als bevorzugte Katalysatoren seien beispielhaft folgende Verbindungen genannt: Lithiumbutanolat, Aluminiumacetylacetonat, Zinkacetylacetonat, Titantetrabutanolat und Zirkontetrabutylat.

Die Vermischung der Eduktströme erfolgt im erfindungsgemäßen Verfahren in einer geeigneten speziellen Mischeinrichtung, die sich durch geringe Mischzeiten auszeichnet.

Die Mischzeit in dieser Mischeinrichtung beträgt üblicherweise von 0,0001 s bis 2 s, bevorzugt von 0,0005 bis 1 s, besonders bevorzugt von 0,001 bis 0,5 s, ganz besonders bevorzugt von 0,005 bis 0,2 s und insbesondere von 0,007 bis 0,1 s. Als Mischzeit ist in dieser Schrift diejenige Zeit zu verstehen, die von dem Beginn des Mischvorgangs vergeht, bis 95,5 % der Fluidelemente des erhaltenen Gemisches einen Mischungsbruch haben, der bezogen auf den Wert des theoretischen Endwert des Mischungsbruchs des erhaltenen Gemisches beim Erreichen des Zustandes perfekter Mischung weniger als 2 % von diesem Endwert des Mischungbruches abweichen (zum Konzept des Mischungsbruches siehe z.B. J.Warnatz, U.Maas, R.W. Dibble: Verbrennung, Springer Verlag, Berlin Heidelberg New York, 1997, 2. Auflage, S. 134.).

Als Mischeinrichtung bevorzugt wird ein Mischkreis, ein Rührbehälter, Rotor-Stator-Systeme, eine Mischpumpe oder eine Düsenmischeinrichtung, beispielsweise Querstrom-, Kammer-, Koaxialmischdüsen, Y- oder T-Mischer, oder eine Vortex-Impinging-Jet-Mischkonfiguration eingesetzt, bevorzugt ein Mischkreis, ein Rührbehälter, eine Mischpumpe oder eine Düsenmischeinrichtung.

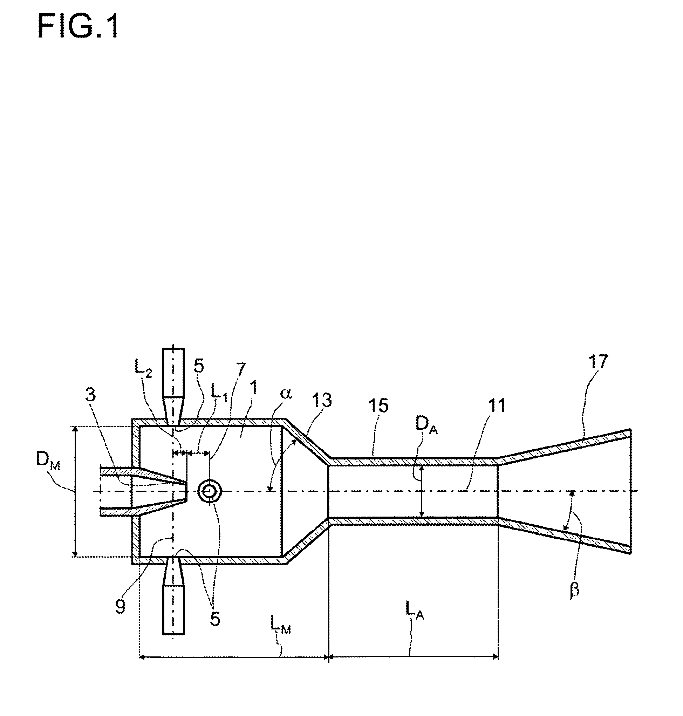

In Figur 1 sei eine Kammerdüse dargestellt, wie sie bevorzugte in der Stufe (a) eingesetzt wird.

Darin bedeuten die Bezugszeichen

| | |
|---|---|
| 1 | Mischkammer |
| 3 | Öffnung koaxial zur Mischkammer |
| 5 | Zufuhröffnung |
| 7 | erste Ebene |
| 9 | zweite Ebene |
| 11 | Achse |
| 13 | Durchmesserverengung |
| 15 | Zone konstanten Durchmessers |
| 17 | Querschnittserweiterung |
| D_{A} | Durchmesser der Zone 15 konstanten Durchmessers |
| D_{M} | Durchmesser der Mischkammer 1 |
| L_{A} | Länge der Zone 15 konstanten Durchmessers |
| L_{M} | Länge der Mischkammer 1 |
| L₁ | Abstand der ersten Ebene 7 zur Öffnung 3 |
| L₂ | Abstand der zweiten Ebene 9 zur Öffnung 3 |
| α | Winkel in dem die Durchmesserverengung 13 ausgeführt ist |
| β | Öffnungswinkel der Querschnittserweiterung 17 |

Eine Vorrichtung zum Mischen des Vorgemischs mit Harnstoff umfasst eine Mischkammer 1, in die Harnstoff und Vorgemisch zugeführt werden. Durch eine Öffnung 3, die koaxial zur Mischkammer 1 angeordnet ist, wird vorzugsweise das Vorgemisch zugegeben. Alternativ ist es jedoch auch möglich, dass durch die koaxial zur Mischkammer angeordnete Öffnung 3 der Harnstoff zugeführt wird. Bevorzugt ist jedoch die Zugabe des Harnstoffs über die koaxial zur Mischkammer angeordnete Öffnung 3. Die koaxial zur Mischkammer 1 angeordnete Öffnung 3 ist z.B., wie hier dargestellt, in Form einer Düse ausgebildet, die in die Mischkammer 1 ragt.

Weiterhin umfasst die Vorrichtung zur Mischung von Harnstoff und Vorgemisch Zufuhröffnungen 5, durch die der Harnstoff bzw. bei Zugabe des Harnstoffs über die koaxial zur Achse der Mischkammer angeordnete Öffnung das Vorgemisch zugegeben wird. Die Zufuhröffnungen 5 sind ebenfalls vorzugsweise als Düsen ausgebildet. Die Zufuhröffnungen 5 sind in mindestens zwei Ebenen 7, 9, die senkrecht zur Achse der Mischkammer angeordnet sind, angeordnet. Die Ebenen 7, 9 sind hier durch gestrichelte Linien dargestellt. In der hier dargestellten Ausführungsform sind die Zufuhröffnungen 5 in zwei Ebenen 7, 9 angeordnet. Eine erste Ebene 7 ist dabei stromab zur koaxial angeordneten Öffnung 3 angeordnet und eine zweite Ebene 9 stromauf.

Neben der hier dargestellten Ausführungsform mit zwei Ebenen 7, 9, in denen die Zufuhröffnungen 5 angeordnet sind, ist es alternativ auch möglich, dass die Zufuhröffnungen in mehr als zwei Ebenen angeordnet sind. In dem Fall, dass die Zufuhröffnungen 5 in mehr als zwei Ebenen 7, 9 angeordnet sind, ist jeweils mindestens eine Ebene stromauf und mindestens eine Ebene stromab der koaxial angeordneten Öffnung 3 angeordnet.

Bevorzugt sind in jeder Ebene 7, 9 zwei Zufuhröffnungen 5 angeordnet, wobei sich die Zufuhröffnungen 5 jeweils diametral gegenüberliegen. Durch die Anordnung, bei der die Zufuhröffnungen 5 diametral gegenüberliegen, treffen sich die Hauptrichtungen der Zufuhröffnungen 5 in der Achse 11 der Mischkammer 1.

Das Verhältnis des Abstandes L₁ der ersten Ebene 7 zur koaxial zur Mischkammer angeordneten Öffnung 3 bezogen auf den Durchmesser D_{M} der Mischkammer 1 liegt vorzugsweise im Bereich von 0 bis 1, mehr bevorzugt im Bereich von 0,01 bis 0,5 und insbesondere im Bereich von 0,05 bis 0,2. Wenn Zufuhröffnungen 5 in mehr als einer Ebene stromab zur koaxial zur Mischkammer angeordneten Öffnung 3 angeordnet sind, so ist dies der Abstand der Ebene, die der koaxial zur Mischkammer angeordneten Öffnung 3 am nächsten liegt.

Das Verhältnis des Abstandes L₂ der zweiten Ebene 9, die stromauf zur koaxial zur Mischkammer 1 angeordneten Öffnung 3 angeordnet ist, bezogen auf den Durchmesser D_{M} der Mischkammer 1 liegt ebenfalls vorzugsweise im Bereich von 0 bis 1, mehr bevorzugt im Bereich von 0,01 bis 0,5 und insbesondere im Bereich von 0,05 bis 0,2. Wenn Zufuhröffnungen 5 in mehr als zwei Ebenen stromauf zur koaxial zur Mischkammer 1 angeordneten Öffnung 3 angeordnet sind, so entspricht dies dem Abstand der Ebene, die der Öffnung 3 am nächsten liegt.

An ihrem stromabwärtigen Ende weist die Mischkammer 1 eine Durchmesserverengung 13 auf. Die Durchmesserverengung 13 ist vorzugsweise konisch ausgebildet und mit einem Winkel α im Bereich von 10 bis 90°, bevorzugt mit einem Winkel im Bereich von 15 bis 60° und besonders bevorzugt mit einem Winkel von 18 bis 40° zur Achse 11 der Mischkammer 1 ausgeführt.

An die Durchmesserverengung 13 schließt sich eine Zone konstanten Durchmessers 15 an. Die Zone 15 konstanten Durchmessers weist einen Durchmesser D_{A} auf, wobei das Verhältnis des Durchmessers D_{A} der Zone 15 konstanten Durchmessers zum Durchmesser D_{M} der Mischkammer 1 wie vorstehend bereits beschrieben im Bereich von 0,2 bis 0,7, mehr bevorzugt im Bereich von 0,25 bis 0,65 und insbesondere im Bereich von 0,3 bis 0,6 liegt. Mit der Durchmesserverengung 13 nimmt der Durchmesser vom Durchmesser D_{M} der Mischkammer 1 auf den Durchmesser D_{A} der Zone 15 konstanten Durchmessers ab.

An die Zone 15 konstanten Durchmessers schließt sich eine Querschnittserweiterung 17 an. Die Querschnittserweiterung 17 ist dabei vorzugsweise in Form eines Diffusors ausgebildet. Die Querschnittserweiterung 17 weist einen Öffnungswinkel β auf, der so ausgewählt ist, dass in der Querschnittserweiterung 17 keine Ablösung der Strömung auftritt. Alternativ zur hier dargestellten Ausführungsform mit einer sich konisch erweiternden Querschnittserweiterung 17 ist es z.B. auch möglich, dass der Durchmesser in der Querschnittserweiterung 17 stufenweise erweitert wird. In diesem Fall ist zwischen den einzelnen Stufen, in denen der Durchmesser erweitert wird, jeweils ein Bereich mit konstantem Durchmesser angeordnet. Alternativ ist es auch möglich, dass zwischen den einzelnen Stufen jeweils ein Bereich ausgebildet ist, in dem sich der Durchmesser konisch erweitert.

Besonders bevorzugt ist die Querschnittserweiterung 17 jedoch konisch ausgebildet und der Öffnungswinkel β der Querschnittserweiterung 17 ist vorzugsweise < 15°, mehr bevorzugt < 10° und besonders bevorzugt < 8°.

Die Länge der Querschnittserweiterung 17 ist bevorzugt so gewählt, dass sich der Durchmesser auf den Durchmesser des sich an die Vorrichtung zur Vermischung des Vorgemischs und Harnstoff anschließenden Reaktors in Stufe (b) erweitert, der hier nicht dargestellt ist.

Um eine kurze Verweilzeit und hohe Mischgeschwindigkeiten in der Mischkammer 1 zu erzielen, ist das Verhältnis der Länge L_{M} der Mischkammer 1 bezogen auf den Durchmesser D_{M} vorzugsweise im Bereich zwischen 1 und 2 und insbesondere im Bereich von 1 bis 1,5. Das Verhältnis der Länge L_{A} der Zone 15 konstanten Durchmessers bezogen auf den Durchmesser D_{A} der Zone konstanten Durchmessers liegt vorzugsweise im Bereich von 1 bis 10, mehr bevorzugt im Bereich von 1,5 bis 9 und insbesondere im Bereich von 2 bis 8.

In einer weiteren bevorzugten Ausführungsform der vorliegenden Erfindung wird als Mischeinrichtung in der Stufe (a) eine Koaxialdüse eingesetzt. Mit dieser Koaxialdüse ist es in einer bevorzugten Ausführungsform möglich, durch den Strahl der einen Komponente die zweite anzusaugen, so daß es möglich ist auf die Pumpe zur Förderung der zweiten Komponente zu verzichten.

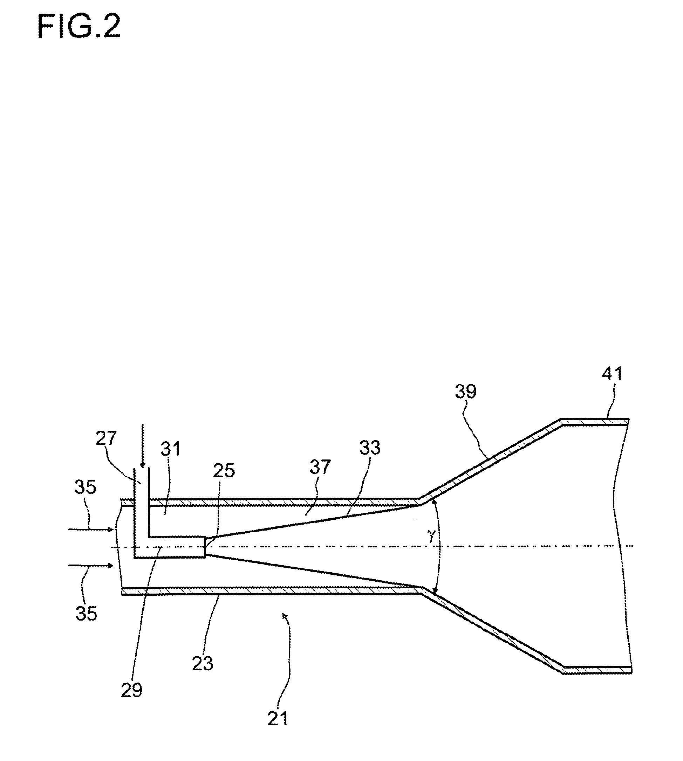

In Figur 2 sei eine solche Koaxialdüse dargestellt.

Darin bedeuten die Bezugszeichen
- 21: Mischeinrichtung
- 23: Rohr
- 25: zentrale Düse
- 27: Zulauf
- 29: Abschnitt des Zulaufs 27
- 31: Ringspalt
- 33: aufweitender Strahl
- 35: Vorgemischzulauf
- 37: Mischzone
- 39: Diffusor
- 41: Reaktor
- γ: Öffnungswinkel

Eine Mischeinrichtung 21 umfasst ein Rohr 23, in das eine zentrale Düse 25 mündet. Das Rohr 23 umschließt einen Zulauf 27 zur zentralen Düse 25. Der Zulauf 27 ist rohrförmig gestaltet und weist einen Abschnitt 29 auf, der entlang der Achse des Rohres 23 verläuft. Auf diese Weise wird zwischen dem Abschnitt 29 des Zulaufs 27 und dem den Abschnitt 29 des Zulaufs 27 umgebenden Rohr 23 ein Ringspalt 31 ausgebildet.

Über den Zulauf 27 wird der Mischeinrichtung 21 Harnstoff zugeführt. Der Harnstoff tritt aus dem Zulauf 27 über die zentrale Düse 25 besonders dann bevorzugt mit einer hohen Geschwindigkeit aus, wenn die Mischeinrichtung als Ejektor betrieben werden soll.

Der aus der zentralen Düse 25 austretende Harnstoff bildet einen sich aufweitenden Strahl 33, wodurch im Ejektorbetrieb Medium aus der Umgebung mitgerissen wird. Hierdurch entsteht ein Unterdruck im Ringspalt 31. Durch den am Ringspalt 31 entstehenden Unterdruck wird ein Vorgemisch beispielsweise aus einem Vorratsbehälter, der das Vorgemisch enthält, angesaugt, strömt durch den Ringspalt 31 und gelangt in eine Mischzone 37, die sich an die zentrale Düse 25 im Rohr 23 anschließt. Der Zulauf des Vorgemischs ist in der Figur durch Pfeile 35 dargestellt.

Selbstverständiich kann die Mischeinrichtung aber auch so betrieben werden, daß das Vorgemisch aktiv, beispielsweise mit einer Pumpe gefördert wird (Strom 35). In diesem Fall kann der Harnstoff 25 mit einer hohen Geschwindigkeit gefördert werden, dies ist jedoch für den Betrieb der Mischeinrichtung nicht zwingend erforderlich.

In der Mischzone 37, die sich an die zentrale Düse 25 im Rohr 23 anschließt, vermischen sich der aus der zentralen Düse 25 ausströmende Harnstoff und das vom Harnstoff aus dem Ringspalt 31 mitgerissene Vorgemisch zu einem Gemisch. Aufgrund der hohen Geschwindigkeit des Harnstoffs ist die Strömung in der Mischzone 37 turbulent und es erfolgt eine schnelle Vermischung von Vorgemisch und Harnstoff.

In einer bevorzugten Ausführungsform ist der Rohrdurchmesser in der Mischzone 37 kleiner ausgeführt als der Rohrdurchmesser vor der Mischstelle, beispielsweise auf Höhe der zentralen Düse 25. In einer besonders bevorzugten Ausführungsform verjüngt sich der Rohrdurchmesser zur Mischstelle konisch.

In einer weiteren bevorzugten Ausführungsform weist die zentrale Düse 25 einen geringeren Öffnungsdurchmesser auf als das Zuleitungsrohr 29.

An die Mischzone 37 schließt sich ein Diffusor 39 an. Im Diffusor 39 nimmt der Strömungsquerschnitt im Allgemeinen konstant zu. Hierzu weist der Diffusor 39 einen Öffnungswinkel γ im Bereich von 4° bis 20°, bevorzugt von 6° bis 16 ° auf. Es ist aber auch möglich, daß der Öffnungswinkel γ zunächst hohe (etwa 15 bis 25°) und anschließend stetig abnehmende Werte (etwa 4 bis 10°) annimmt (Glockendiffusoren).

Im Diffusor 39 nimmt die Geschwindigkeit des Reaktionsgemisches ab, gleichzeitig steigt der Druck.

An den Diffusor 39 schließt sich der Reaktor 41 in der Stufe (b) an.

Anstelle der hier beschriebenen Zuführung der Ströme (Dosierung des Harnstoffs durch Leitung 25 und der Vormischung durch Leitung 35) kann die Dosierung alternativ auch vertauscht erfolgen, daß der Harnstoff durch Leitung 35 und die Vormischung über Leitung 25 eindosiert werden.

Wenn die Düse nicht als Ejektor betrieben wird, liegt der Druck in den Zuleitungen zur Düse erheblich höher als im Ausgang der Mischdüse, üblicherweise jedoch nicht höher als 110 bar abs, bevorzugt nicht höher als 100 bar abs, besonders bevorzugt beträgt der Druck von 5 bis 95 bar abs, ganz besonders bevorzugt von 10 bis 50 bar abs und insbesondere von 10 bis 30 bar abs.

Der Druck am Ausgang der Mischeinrichtung liegt in der Regel oberhalb des Reaktionsdrucks in Stufe b), beispielsweise zwischen 5 und 100 bar, bevorzugt zwischen 10 und 80 bar, besonders bevorzugt zwischen 10 und 50 bar.

Die Temperatur des Austrages aus der Mischeinrichtung beträgt in der Regel zwischen 20 und 250 °C, bevorzugt 40 - 220 und besonders bevorzugt 100 - 180 °C.

Bei der Verwendung eines Mischkreises oder eines Rührbehälters als Mischeinrichtung ist es wichtig, dass die Reaktanden mit hoher Geschwindigkeit eingedüst wird. Üblicherweise liegen die Geschwindigkeiten zwischen 10 und 100 m/s, bevorzugt zwischen 20 und 80 m/s.

Bevorzugt wird eine Mischdüse und eine Mischpumpe als Mischeinrichtung eingesetzt. Besonders bevorzugt wird als Mischeinrichtung eine Mischdüse verwendet. Hierbei ist es wichtig, dass sowohl der Harnstoff- als auch der Vormischungsstrom mit hoher Geschwindigkeit in die Mischdüse eingeleitet werden. Die Geschwindigkeiten betragen zwischen 10 und 100 m/s, bevorzugt zwischen 20 und 80 m/s.

Durch die erfindungsgemäße Vormischung von Alkohol und Amin ist der Umsatz der Reaktion des Amins mit Harnstoff zum Diharnstoff bezogen auf Aminogruppen im eingesetzten Amin in der Stufe a) deutlich höher als im vergleichbaren Verfahren gemäß der WO 2007/31444.

### b) Reaktion des Gemisches aus a)

Die die Mischeinrichtung verlassende Flüssigphase wird nun mindestens einem, bevorzugt genau einem Rohrreaktor oder einer Kaskade mehrerer rückvermischter Reaktoren, die von ihrer Verweilzeitverteilung einem Rohrreaktor ähneln, zugeführt.

Der Reaktor schließt sich bevorzugt direkt an die Mischeinrichtung (a) an.

Ein Rohrreaktor sollte bevorzugt weitestgehend rückvermischungsfrei sein. Dies wird beispielsweise erreicht durch das Verhältnis des Durchmessers des Rohrreaktors zu dessen Länge oder durch Einbauten, wie Lochböden, Schlitzböden oder statische Mischer. Bevorzugt wird die Rückvermischungsfreiheit durch das Verhältnis von Länge zu Durchmesser des Rohrreaktors erreicht.

Als Rohrreaktor eignen sich beispielsweise solche Rohre, deren Längen- zu Durchmesserverhältnis größer als 5 ist, bevorzugt größer als 6, besonders bevorzugt größer als 10.

Die Bodensteinzahl des Rohrreaktors sollte größer als 5 sein, bevorzugt größer als 6, besonders bevorzugt größer als 10, ganz besonders bevorzugt von 10 bis 600 und insbesondere von 10 bis 100.

Ein Aspekt, der wesentlich zur Erfindung beiträgt ist das Vorliegen einer Strömung, die idealerweise eine Pfropfenströmung (Kolbenströmung, plug-flow) ist und dieser in der Realität so weit wie erforderlich angenähert werden soll. Dazu wird die axiale Durchmischung, also eine Durchmischung entlang der Flußrichtung durch den Reaktor, möglichst verringert und die Strömung ist idealerweise turbulent.

Dies wird in der Praxis erreicht durch hohe Strömungsgeschwindigkeiten und geringe Querschnittsflächen beispielsweise in Strömungsrohren.

Der Rohrreaktor kann eine beliebige Orientierung im Raum aufweisen. Bevorzugt wird er als senkrechter Rohrreaktor aufgebaut, der besonders bevorzugt von unten nach oben durchströmt wird.

Der Rohrreaktor kann isotherm oder bevorzugt temperiert ausgeführt werden. Eine Temperierung kann durch eine Mantelheizung oder durch innenliegende Rohre oder Platten erfolgen. Die Beheizung erfolgt bevorzugt durch den Mantel.

Selbstverständlich kann der Rohrreaktor auch aus mehreren seriell geschalteten Rohrstücken bestehen, solange die Rückvermischungsfreiheit gewährleistet bleibt. Falls erforderlich können optional im Verlauf des Rohrreaktors, beispielsweise zwischen derartige Rohrstücke Phasenscheider zur Trennung von flüssiger und gasförmiger Phase vorgesehen werden, in denen während der Reaktion entstandener Ammoniak abgetrennt werden kann, so daß das Gleichgewicht der Reaktion verschoben wird.

Zur Vergrößerung der Produktionskapazität können erfindungsgemäß auch mehrere Rohrreaktoren parallel geschalten werden.

Gegebenenfalls kann in den Rohrreaktor, wie oben ausgeführt, an einer oder mehreren Stellen, beispielsweise am Anfang und in der Mitte des Rohrreaktors, noch Harnstoff und/oder Alkohol oder bevorzugt Amin nachdosiert werden.

Die mittlere Verweilzeit im Rohrreaktor beträgt in der Regel 10 Sekunden bis 5 Stunden, bevorzugt 20 Sekunden bis 20 Minuten, besonders bevorzugt 30 Sekunden bis 10 Minuten.

Um die Gasbelastung für die Folgestufe gering zu halten kann der Austrag aus dem Rohrreaktor in einer bevorzugten Ausführungsform einem Phasenscheider zugeführt werden und die dem Phasenscheider entnommene Flüssigphase dann der Folgestufe zugeführt werden.

Ein solcher Phasenscheider ist ein Behälter, in dem die Phasentrennung zwischen Gas- und Flüssigphase durch die Beruhigung der zweiphasigen, aus dem Gleichstromreaktor austretenden Strömung und Wirkung der Erdschwere erreicht wird. Alternativ ist auch eine Trennung der beiden Phasen im Zentrifugalfeld, z.B. mittels eines Zyklons, möglich.

Der Phasenscheider kann isotherm oder bevorzugt beheizt ausgeführt werden, um das Ausfallen schwer löslicher Nebenprodukte zu verhindern. Die Beheizung kann beispielsweise über den Mantel oder über einen Kreislauf mit einem externen Wärmetauscher erfolgen. Bei Verwendung eines externen Wärmetauschers reicht eine normale Isolierung des Wärmetauschers.

Die Temperatur im Rohrreaktor und im gegebenenfalls vorhandenen Phasenscheider beträgt im allgemeinen zwischen 50 °C und 300 °C, bevorzugt zwischen 180 °C und 220 °C.

Der Druck in Stufe b) beträgt in der Regel zwischen 0,1 bar abs und 30 bar abs und bevorzugt zwischen 5 und 20 bar abs.

Das Überführen des Reaktionsaustrages aus der Stufe b) in die nachfolgende Stufe kann vorteilhaft über Druckhalteventile erfolgen, wobei der Druck in Stufe b) in der Regel mindestens 0,1 bar oberhalb des in Stufe c) herrschenden Druckes betragen sollte. Ist dies nicht der Fall, kann das Überführen z.B. mit Hilfe einer Pumpe oder barometrisch erfolgen.

Die Verweilzeit in Stufe b) ist so gewählt, daß der Umsatz, bezogen auf Aminogruppen im eingesetzten Amin zu Urethangruppen, nach Verlassen des Rohrreaktors mindestens 95%, bevorzugt mindestens 98, besonders bevorzugt mindestens 99 und ganz besonders bevorzugt mindestens 99,5% beträgt.

Üblicherweise beträgt die gesamte Verweilzeit in Stufe a) und b) zusammen weniger als 5 Stunden, bevorzugt weniger als 4 Stunden und besonders bevorzugt weniger als 3 Stunden.

Der Austrag der Reaktionsmischung aus (b) kann bei vollständigem Umsatz der Amine zum Urethan direkt der Ammoniakabtrennung (c) zugeführt werden oder er wird zur Erzielung eines vollständigen Umsatzes einem weiteren Reaktor oder Reaktorsystem zugeführt. Als Reaktoren können weitere Rohrreaktoren, Mischreaktorkaskaden oder Kolonnen mit der notwendigen mittleren Verweilzeit zum Einsatz kommen.

Ist der Umsatz, bezogen auf Aminogruppen im eingesetzten Amin zu Urethangruppen, nach Verlassen des Rohrreaktors noch nicht vollständig und beträgt beispielsweise weniger als 95%, so kann der Austrag nochmals nachreagiert werden.

Dazu kann das Reaktionsgemisch zur Vervollständigung des Umsatzes in einem weiteren Rohrreaktor oder aber auch in einem rückvermischten Reaktor nachreagieren gelassen werden, bevorzugt bis der Umsatz 98% oder mehr beträgt.

Unter rückvermischtem Reaktorsystem wird hier verstanden, dass die Bodensteinzahl des Reaktorsystems kleiner 5, bevorzugt kleiner 4, ist.

### c) Ammoniakabtrennung

Zur Abtrennung des Ammoniaks werden zweckmäßigerweise Kolonnen verwendet, bevorzugt wird der Ammonik per Destillation abgetrennt. Dadurch gelingt eine gute Trennung zwischen dem Alkohol und Ammoniak. Üblicherweise erfolgt die Abtrennung in einem Druckbereich von 0,01 - 20 bar, vorzugsweise bei 0,04 - 15 bar. Die notwendigen Temperaturen richten sich nach dem verwendeten Alkohol bzw. dem Alkoholgemisch. Für n-Butanol liegt die Temperatur beispielsweise bei 60 - 150 °C, bevorzugt bei 80 bis 140 °C.

Es hat sich als vorteilhaft erwiesen, das entstehende Ammoniak sofort aus der Reaktionsmischung abzutrennen, so daß eine Belegung durch Ammoniumcarbaminat, welches in minimalen Mengen aus Ammoniak und Kohlendioxid durch Zersetzung von Harnstoff gebildet wird, vermieden werden kann.

Diese Destillationseinheit ist von an sich bekannter Bauart und weist die üblichen Einbauten auf. Als Kolonneneinbauten kommen prinzipiell alle gängigen Einbauten in Betracht, beispielsweise Böden, Packungen und/oder Schüttungen. Von den Böden sind Glockenböden, Siebböden, Ventilböden, Thormannböden und/oder Dual-Flow-Böden bevorzugt, von den Schüttungen sind solche mit Ringen, Wendeln, Sattelkörpem, Raschig-, Intos- oder Pall-Ringen, Barrel- oder Intalox-Sätteln, Top-Pak etc. oder Geflechten bevorzugt. Bevorzugt werden Böden verwendet, besonders bevorzugt Glockenböden.

Die Destillationskolonne weist bevorzugt 10 - 20 theoretische Trennböden auf.

### d) Abtrennung des überschüssigen Alkohols

Aus der erhaltenen ammoniakabgereicherten Reaktionsmischung werden dann Alkohol, Dialkylcarbonate, sofern solche gebildet wurden oder in der Reaktionsmischung vorliegen, oder Carbamidsäurealkylester oder Mischungen aus mindestens zwei dieser Komponenten abgetrennt und vorzugsweise in die Reaktionsstufe (0) und/oder (a) und/oder (b) zurückgeführt.

Zur Abtrennung der Komponenten wird die Reaktionsmischung vorteilhafterweise vom Druckniveau der Reaktionsstufe (b) auf einen Druck im Bereich von 1 bis 500 mbar, vorzugsweise von 10 bis 100 mbar entspannt. Man erhält hierbei gasförmige Brüden, die die überwiegende Alkoholmenge sowie 0 bis 30 Gew.%, vorzugsweise 1 bis 10 Gew.% Dialkylcarbonat und/oder 1 bis 50 Gew.%, vorzugsweise 1 bis 20 Gew.% Carbamidsäurealkylester enthalten, und einen flüssigen Austrag, der im wesentlichen aus dem monomeren Polyurethan, vorzugsweise Diurethan, besteht und gegebenenfalls Oligoharnstoff-polyurethanen und hochsiedende Oligomere enthält.

Die erhaltenen Brüden (d_{L}) werden in nachfolgenden zweckmäßigerweise destillativen Reinigungsstufen, vorzugsweise durch Rektifikation, getrennt und die hierbei isolierten Wertprodukte Alkohol und Carbamidsäurealkylester, einzeln oder als Mischung, vorzugsweise in die Reaktionsstufe (a) zur Bildung der monomeren Polyurethane zurückgeführt.

Zur destillativen Abtrennung des Alkohols oder des Alkoholgemisches wird häufig ein sogenannter Flash eingesetzt. Dieser Apparat kann ein Behälter oder eine Kombination von Behälter und Kolonne vorzugsweise eine Kolonne sein, wobei im Kopf der Alkohol bzw. das Alkoholgemisch und im Sumpf das Urethan abgezogen werden kann. Im Kopf der Kolonne können neben dem Alkohol auch weitere leichter als das Urethan siedende Stoffe enthalten sein. Die Trennung erfolgt in einem Druckbereich von 0,001 bis 1 bar, vorzugsweise bei 0,02 - 0,5 bar.

### e) Urethanaufreinigung

Die in der Reaktionsstufe (d) nach Abtrennung der Brüden in der Regel als Sumpfaustrag erhaltene flüssige, die monomeren Polyurethane, vorzugsweise Diurethane, und gegebenenfalls Oligoharnstoff-polyurethane und hochsiedende Oligomere enthaltende Reaktionsmischung (d) kann entweder vollständig in die Folgestufe geführt werden oder wird bevorzugt in zwei Teilströme geteilt, wobei das Gewichtsverhältnis der Teilmengen 5 bis 50:95 bis 50 Gew.-Teile, vorzugsweise 10 bis 30:90 bis 70 Gew.-Teile beträgt.

Die gleich große oder vorzugsweise kleinere Teilmenge wird destillativ getrennt mittels einer üblichen Destillationsanlage, vorzugsweise eines Dünnschichtverdampfers, bei einer Temperatur von 170 bis 240°C, vorzugsweise von 180 bis 230°C und unter einem Druck von 0,001 - 1 bar, vorzugsweise 0,002 - 0,01 bar, in ein Wertprodukt, das die Polyurethane, vorzugsweise Diurethane und die leichter siedende Nebenprodukte enthält (e_{L}), und nicht destillierbare Nebenprodukte (e_{H}), die aus dem Herstellungsverfahren abgetrennt und üblicherweise als nicht verwertbarer Rückstand verworfen werden. Das Wertprodukt (Destillat) wird mit der gleich großen oder vorzugsweise größeren anderen Teilmenge vereinigt und die vereinigte, Polyurethane, vorzugsweise Diurethane enthaltende Reaktionsmischung der thermischen Spaltung (f) zugeführt.

Durch diese Verfahrensmaßnahme in der Reaktionsstufe (e) wird der Anteil an nicht destillierbaren Nebenprodukten in der Reaktionsmischung, die sich bei den nacheinander ablaufenden Teilreaktionen bilden und durch die Rückführung verwertbarer Einsatzstoff im Reaktionskreislauf ständig anreichern würden, auf einen Gehalt von 3 bis 30 Gew.%, vorzugsweise 5 bis 20 Gew.% begrenzt und dadurch eine mit hoher Selektivität störungsfrei ablaufende Reaktion gewährleistet.

Als Destillationseinrichtungen können Dünnschichtverdampfer oder Kurzwegverdampfer zum Einsatz kommen. Das Urethan wird bei Drücken von 0,001 - 1 bar, vorzugsweise im Bereich von 0,002 - 0,01 bar destilliert. Das Destillat (e_{L}) wird der Spaltung (f) zugeführt.

Der hochsiederhaltige Sumpf (es) wird bevorzugt verworfen oder kann weniger bevorzugt teilweise der Reurethanisierung (h) zugeführt werden. Denkbar ist es auch, diesen hochsiederhaltigen Sumpf zur Rückführung verwertbarer Spaltprodukte beispielsweise einem Schaufeltrockner zuzuführen, wie beschrieben in der WO 2007/036479.

### f) Urethanspaltung

Die in der Reaktionsstufe (e) erhaltene, Polyurethane, vorzugsweise Diurethane, enthaltende Reaktionsmischung wird in einer geeigneten Vorrichtung, bevorzugt lösungsmittelfrei in flüssiger Phase in Gegenwart von Katalysatoren bei Temperaturen von 200 bis 300°C, vorzugsweise 220 bis 280°C und unter vermindertem Druck von 0,01 - 0,6 bar, vorzugsweise im Bereich von 0,02 - 0,1 bar kontinuierlich thermisch gespalten. Der Umsatz von Polyurethan zu Polyisocyanat, vorzugsweise von Diurethan zu Diisocyanat, in der Vorrichtung zur thermischen Spaltung kann in Abhängigkeit vom verwendeten Polyurethan weitgehend frei gewählt werden und liegt zweckmäßigerweise in einem Bereich von 10 bis 98 Gew.%, vorzugsweise 40 bis 90 Gew.% der zugeführten Polyurethanmenge.

Der ungespaltene Anteil der Reaktionsmischung, der nicht umgesetzte Polyurethane, Oligoharnstoff-polyurethane, hochsiedende Oligomere und andere wieder verwertbare und unverwertbare Nebenprodukte enthält, wird abgetrennt, kontinuierlich aus der Spaltvorrichtung ausgeschleust (f_{H}) und direkt oder gegebenenfalls nach Umsetzung mit Alkohol in der Reurethanisierung (h) in die Reaktionsstufe (a) und/oder (b) zurückgeführt.

Als Katalysatoren zur chemischen Spaltung der Polyurethane finden z.B. die vorgenannten, die Urethanbildung katalysierende anorganischen und organischen Verbindungen Verwendung.

Besonders bewährt und daher vorzugsweise verwendet werden Dibutylzinndilaurat, Eisen-(III)-acetylacetonat, Kobalt-(II)-acetylacetonat, Zinkacetylacetonat, Zirkon tetra-n-butanolat und Zinn-(II)-dioctoat.

Als Spaltvorrichtungen eignen sich beispielsweise zylinderförmige Spaltreaktoren, wie z.B. Röhrenöfen oder vorzugsweise Verdampfer, beispielsweise Dünnschicht- oder Bulkverdampfer, wie z.B. Robertverdampfer, Herbertverdampfer, caddle-typ-Verdampfer, Plattenspalter und vorzugsweise Heizkerzenverdampfer.

Die Trennung der Spaltprodukte erfolgt in einer Kolonne, bei der üblicherweise das Isocyanat in der Seite (f_{M}) und der Alkohol (f_{L}) am Kopf abgezogen werden.

### g) Isocyanatreinigung

Das Rohisocyanatgemisch wird in einer sich anschließenden Destillation von Rekombinationsprodukten, Nebenprodukten und sofern vorhanden dem Lösungsmittel befreit. Die Nebenprodukte werden vorzugsweise in die thermische Spaltung zurückgeführt. Ein Teil kann auch ausgeschleust werden.

Die bei der thermischen Spaltung gebildeten Spaltprodukte, die sich vor allem aus Alkohol, Polyisocyanat, vorzugsweise Diisocyanat, und partiell gespaltenen Polyurethanen zusammensetzen, werden danach vorteilhafterweise mit Hilfe einer oder mehrerer Destillationskolonnen, vorzugsweise durch Rektifikation bei Temperaturen von 100 bis 220°C, vorzugsweise 120 bis 170°C und einem Druck von 1 bis 200 mbar, vorzugsweise 5 bis 50 mbar, in Leichtsieder und besonders Alkohol (g_{L}) und eine rohe Polyisocyanatmischung (g_{M}) mit einem Polyisocyanatgehalt von 85 bis 99 Gew.%, vorzugsweise von 95 bis 99 Gew.% getrennt. Die bei der destillativen Trennung anfallenden höhersiedenden Nebenprodukte (g_{H}) und insbesondere die ungespaltenen und partiell gespaltenen Polyurethane werden vorzugsweise in die Spaltvorrichtung (f) und/oder Reurethanisierung (h) geführt.

Mit dem Index "L" werden hier leichtsiedende Ströme der einzelnen Stufen gekennzeichnet, mit dem Index "H" hochsiedende und mit "M" mittelsiedende.

Die vorzugsweise durch Rektifikation erhaltene rohe Polyisocyanatmischung (g_{M}) wird durch Destillation bei einer Temperatur von 100 bis 180°C und unter einem Druck von 1 bis 50 mbar gereinigt, wobei die einzelnen Fraktionen zurückgeführt oder als Reinprodukt isoliert werden. Wie bereits ausgeführt wurde, wird bei der bevorzugt angewandten Reindestillation die Kopffraktion, die vorzugsweise aus Polyisocyanat, insbesondere Diisocyanat besteht, gegebenenfalls nach Umsetzung der freien Isocyanatgruppen mit Alkohol in die Reaktionsstufe (a) und/oder (b) zurückgeführt, die Seitenfraktion, die aus reinem Polyisocyanat, insbesondere Diisocyanat, vorzugsweise mit einer Reinheit von mindestens 98 Gew.%, insbesondere über 99 Gew.% besteht, wird abgeleitet und der Lagerung zugeführt und die Sumpffraktion, die als wesentliche Komponenten die partiell gespaltenen Polyurethane und Polyisocyanate enthält, wird vorzugsweise in die Spaltvorrichtung zur thermischen Spaltung zurückgeführt.

Nach anderen Verfahrensvarianten kann die Sumpffraktion (g_{H}) jedoch auch in die Destillationskolonne (d) zur Trennung von rohem Polyisocyanat und Alkohol oder in die Reaktionsstufe (a) und/oder (b), die Polyurethanbildung, zurückgeführt werden. Möglich ist auch eine Teilung der Sumpffraktion in 2 oder 3 Produktströme, wobei diese vorzugsweise in der Polyurethanbildung (a) und die Spaltvorrichtung (f) sowie gegebenenfalls in die Destillationskolonne (g) oder in die Reurethanisierung (h) zurückgeführt werden.

h) Die Umsetzung des Reaktionsaustrages (f_{H}) aus f) und/oder Destillationsrückstände (g_{H}) aus (g) werden vorzugsweise erneut dem Prozess zugeführt. Dabei werden mit Alkohol die in diesem Gemisch enthaltenen Isocyanatgruppen und/oder Allophanate und/oder Harnstoffe oder sonstige reaktive Bestandteile zu Urethanen umgewandelt. Es besteht die Möglichkeit, diese Reaktionen in separaten Reaktoren wie z. B. Mischreaktoren oder Strömungsrohren oder auch in (b) durchzuführen. Für die Alkoholyse der Rückstände sind Temperaturen von 100 - 250 °C, vorzugsweise 150 - 220 °C erforderlich. Die mittleren Verweilzeiten liegen dabei im Bereich von wenigen Minuten bis Stunden.

Dazu können beispielsweise die Ströme (f_{H}) und/oder (g_{H}) sowie gegebenenfalls ein Teil des Stromes (e_{H}) mit Alkohol zusammengeführt werden, wobei das Molverhältnis von NCO-Gruppen bzw. deren Äquivalenten, also beispielsweise Urethangruppen, zu Hydroxygruppen bis zu 1:100, bevorzugt bis zu 1:20, besonders bevorzugt bis zu 1:10 beträgt.

Der Alkohol kann dabei beispielsweise der leichtsiedende Strom (dL) aus der Stufe (d) sein und/oder der alkohohaltige Strom (f_{L}) aus der Urethanspaltung (f) und/oder auch frischer Alkohol sein.

Die Reaktionsmischung wird in Gegenwart oder Abwesenheit von Katalysatoren innerhalb von 1 bis 150 min, bevorzugt 3 bis 60 min bei Temperatur von 20 bis 200 °C, bevorzugt 50 bis 170 °C bei einem Druck von 0,5 bis 20 bar, bevorzugt 1 bis 15 bar umgesetzt.

Die Umsetzung kann in einer kontinuierlichen Kesselkaskade oder in einem Rohrreaktor durchgeführt werden.

Als Katalysatoren kommen grundsätzlich alle Verbindungen in Frage, die die Reaktion von NCO- mit OH-Gruppen fördern. Beispielsweise seien genannt Zinnoctoat, Dibutylzinndilaurat, Zinnchlorid, Zinkdichlorid, Zinn-(II)-dioctoat und Triethylamin.

Mit dem erfindungsgemäßen mehrstufigen Verfahren zur kontinuierlichen Herstellung von organischen Polyisocyanaten unter Rückführung und Ausschleusung der Nebenprodukte können destillierbare Polyisocyanate, vorzugsweise Diisocyanat mit hoher Selektivität in sehr guten Ausbeuten hergestellt werden.

Das erfindungsgemäße Verfahren eignet sich insbesondere zur Herstellung von aliphatischen Diisocyanaten, wie 2-Methylpentan-diisocyanat-1,5, isomeren aliphatischen Diisocyanaten mit 6 Kohlenstoffatomen im Alkylenrest und deren Gemische und vorzugsweise Hexamethylendiisocyanat-1,6 und cycloaliphatischen Diisocyanaten, insbesondere 3-Isocyanatomethyl-3,5,5-trimethylcyclohexylisocyanat nach einer wirtschaftlichen Methode.

Die hergestellten Polyisocyanate eignen sich vorzüglich zur Herstellung von Urethan-, Isocyanurat-, Amid- und/oder Harnstoffgruppen enthaltenden Kunststoffen nach dem Polyisocyanat-Polyadditionsverfahren. Sie finden ferner Verwendung zur Herstellung von mit Urethan-, Biuret- und/oder Isocyanuratgruppen modifizierten Polyisocyanatmischungen. Derartige Polyisocyanatmischungen aus aliphatischen oder cycloaliphatischen Diisocyanaten werden insbesondere zur Herstellung von lichtbeständigen Polyurethanlacken und -überzügen verwendet.

Nach einer bevorzugten Ausführungsform wird die bei der destillativen Reinigung des rohen Polyisocyanats (f) anfallende Kopffraktion in die Reaktionsstufe (a) zurückgeführt, die Seitenfraktion, die aus im wesentlichen reinem Polyisocyanat besteht, wird einem Behälter zur Lagerung und die Sumpffraktion in die Reaktionsstufe (a) oder (d) oder (a) und (d) zurückgeführt.

Die folgenden Beispiele sollen die Erfindung erläutern, aber nicht auf diese Beispiele einschränken.

### Beispiel

Vergleichsbeispiel gemäß WO 2007/031444 A1:
In einem Behälter (A) wurde 1,6-Hexamethylendiamin bei einer Temperatur von 80 °C vorgelegt. In einem zweiten Behälter wurde n-Butanol (B) gefüllt. In einem Reaktionsmischer (Reaktionsmischer HMR der Firma K-Engineering, Reaktionsvolumen 5 ml, 4000 min-1) wurde mittels Pumpen ein Strom von 0,9 kg/h Amin, 8 kg/h Butanol und 1,8 kg/h flüssiger Harnstoff (C) zugeführt. Die Ströme wurden mit Wärmeüberträgern so vorgewärmt, dass in der Düse (D) mindestens eine Temperatur von 130 °C bis 150 °C vorlag.

Das Reaktionsgemisch durchströmte dann einen mantelbeheizten Rohrreaktor (E) mit einer Länge von 6 m (Bodensteinzahl ca. 40), einer mittleren Verweilzeit von wenigen Sekunden und einer Reynoldszahl von ca. 10000.

Das Reaktionsgemisch aus dem Strömungsrohr wird danach in einem Mischreaktor (F) bis zum vollständigen Umsatz des Amins mit einer mittleren Verweilzeit von ca. 4 Stunden verweilen gelassen. Die Temperatur im Reaktor beträgt bei einem Druck von 11 bar ca. 215 °C. Der Reaktionsaustrag enthält ca. 2,4 kg/h Hexamethylendibutylurethan und kann wie bekannt zum 1,6-Hexamethylendiisocyanat gespalten und aufgearbeitet werden.

### Beispiel 1

In einem Behälter wurde 1,6-Hexamethylendiamin bei 80 °C vorgelegt. In zwei weiteren Behältern wurde n-Butanol bei Raumtemperatur und Harnstoff bei 135 °C vorgelegt. Der Harnstoff wurde dabei in einem Druckautoklaven als Feststoff eingefüllt und geschmolzen. In einem Wärmetauscher wurde der Butanolstrom vorgewärmt. In einer sich anschließenden Mischeinrichtung gemäß Fig. 1 wurde dieser Strom von 4,5 kg/h n-Butanol mit dem Aminstrom (0,43 kg/h Diamin) vermischt. Die sich einstellende Mischtemperatur reichte aus, um diesen Mischstrom in einer weiteren Mischeinrichtung mit dem flüssigen Harnstoffstrom (0,59 kg/h) auf 200 °C zu erwärmen. Die weitere Reaktorkonfiguration und die Prozeßparameter entsprechen denen des Vergleichsbeispieles.

Die erreichten Ausbeuten liegen etwas höher als im Vergleichsbeispiel.

## Patentansprüche

1. Verfahren zur Herstellung von Isocyanaten durch Umsetzung mindestens eines Amins mit Harnstoff und mindestens einem Alkohol zum korrespondierenden Urethan in mindestens einer Mischeinrichtung mit mindestens einem angeschlossenem Verweilzeitreaktor und anschließender Spaltung der so erhaltenen Urethane in die korrespondierenden Isocyanate, **dadurch gekennzeichnet, daß** man zumindest einen Teil des Amins und zumindest einen Teil des mindestens einen Alkohols in Form eines Gemisches in der mindestens einen Mischeinrichtung mit Harnstoff sowie dem gegebenenfalls verbliebenen restlichen Amin sowie dem gegebenenfalls verbliebenen restlichen Alkohol vermischt und reagieren läßt.

2. Verfahren gemäß Anspruch 1, **dadurch gekennzeichnet, daß** mindestens 95,5 % der Fluidelemente des Gemisches aus zumindest einem Teil des Amins und zumindest einem Teil des mindestens einen Alkohols einen Mischungsbruch haben, der bezogen auf den Wert des theoretischen Endwerts des Mischungsbruches des erhaltenen Gemisches beim Erreichen des Zustandes perfekter Mischung weniger als 2 % von diesem Endwert des Mischungs bruches abweichen.

3. Verfahren gemäß Anspruch 1, **dadurch gekennzeichnet, daß** es sich bei der mindestens einen Mischeinrichtung um eine Düsenmischeinrichtung handelt, ausgewählt aus der Gruppe bestehend aus Querstrom-, Kammer-, Ejektor-, Koaxialmischdüsen, Y- und T-Mischer.

4. Verfahren gemäß einem der vorstehenden Ansprüche, **dadurch gekennzeichnet, daß** die Mischzeit in der Mischeinrichtung von 0,0001 s bis 2 s beträgt.

5. Verfahren gemäß einem der vorstehenden Ansprüche, **dadurch gekennzeichnet, daß** Harnstoffstrom und Vormischungsstrom mit einer Geschwindigkeit zwischen 5 und 100 m/s in die Mischvorrichtung eingeleitet werden.

6. Verfahren gemäß einem der vorstehenden Ansprüche, **dadurch gekennzeichnet, daß** das Amin ausgewählt ist aus der Gruppe Butandiamin-1,4, 2-Ethylbutandiamin-1,4, Octandiamin-1,8, Decandiamin-1,10, Dodecandiamin-1,12, Cyclohexandiamin-1,4, 2-Methyl-, 4-Methyl-cyclohexandiamin-1,3, 1,3- und 1,4-Diaminomethylcyclohexan, 2-Methylpentandiamin-1,5, 2,2,4- bzw. 2,4,4-Trimethylhexandiamin-1,6, Hexandiamin-1,6, 3-Aminomethyl-3,5,5-trimethylcyclohexylamin und 1,3- und 1,4-Diaminomethylcyclohexan3 (bzw. 4), 8 (bzw. 9)-Bis(aminomethyl)-tricyclo[5.2.1.0^{2.6}]decan-Isomerengemische.

7. Verfahren gemäß einem der vorstehenden Ansprüche, **dadurch gekennzeichnet, daß** der Alkohol ausgewählt ist aus der Gruppe Methanol, Ethanol, n-Propanol, n-Butanol, iso-Butanol, n-Pentanol, iso-Pentanol, n-Hexanol, Isohexanole, 2-Ethylhexanol, Decanol oder Gemische der genannten Alkohole.

## Claims

1. A process for preparing isocyanates by reaction of at least one amine with urea and at least one alcohol to form the corresponding urethane in at least one mixing apparatus having at least one attached residence reactor and subsequent dissociation of the resulting urethanes into the corresponding isocyanates, wherein at least part of the amine and at least part of the at least one alcohol as a mixture are mixed with urea and any remaining amine and any remaining alcohol in the at least one mixing apparatus and allowed to react.

2. The process according to claim 1, wherein at least 95.5% of the fluid elements of the mixture of at least part of the amine and at least part of the at least one alcohol have a mixing fraction which, based on the value of the theoretical final value of the mixing fraction of the mixture obtained on reaching the state of perfect mixing, deviates by less than 2% from this final value of the mixing fraction.

3. The process according to claim 1, wherein the at least one mixing apparatus is a nozzle mixing apparatus selected from the group consisting of crossflow, chamber, ejector, and coaxial mixing nozzles, and Y- and T-mixers.

4. The process according to any of the preceding claims, wherein the mixing time in the mixing apparatus is from 0.0001 s to 2 s.

5. The process according to any of the preceding claims, wherein the urea stream and the premixture stream are introduced into the mixing apparatus at a velocity in the range from 5 to 100 m/s.

6. The process according to any of the preceding claims, wherein the amine is selected from the group consisting of 1,4-butanediamine, 2-ethyl-1,4-butanediamine, 1,8-octanediamine, 1,10-decanediamine, 1,12-dodecanediamine, 1,4-cyclohexanediamine, 2-methyl- and 4-methyl-1,3-cyclohexanediamine, 1,3- and 1,4-diaminomethylcyclohexane, 2-methyl-1,5-pentanediamine, 2,2,4- and 2,4,4-trimethyl-1,6-hexanediamine, 1,6-hexanediamine, 3-aminomethyl-3,5,5-trimethylcyclohexylamine and 1,3- and 1,4-diaminomethylcyclohexane, 3 (or 4), 8 (or 9) -bis(aminomethyl)tricyclo[5.2.1.0^{2.6}]decane isomer mixtures.

7. The process according to any of the preceding claims, wherein the alcohol is selected from the group consisting of methanol, ethanol, n-propanol, n-butanol, isobutanol, n-pentanol, isopentanol, n-hexanol, isohexanols, 2-ethylhexanol, decanol and mixtures of the alcohols mentioned.

## Revendications

1. Procédé pour la production d'isocyanates par mise en réaction d'au moins une amine avec de l'urée et au moins un alcool, pour l'obtention de l'uréthane correspondant, dans au moins un dispositif de mélange comportant au moins un réacteur raccordé à temps de séjour et coupure subséquente des uréthanes ainsi obtenus en les isocyanates correspondants, **caractérisé en ce qu'**on mélange et fait réagir au moins une partie de l'amine et au moins une partie dudit au moins un alcool sous forme d'un mélange dans ledit au moins un dispositif de mélange, avec l'urée ainsi que l'amine éventuellement restante ainsi que l'alcool résiduel éventuellement restant.

2. Procédé selon la revendication 1, **caractérisé en ce qu'**au moins 95,5 % des éléments fluides du mélange d'au moins une partie de l'amine et d'au moins une partie dudit au moins un alcool ont une lacune de miscibilité qui, par rapport à la valeur de la valeur finale théorique de la lacune de miscibilité du mélange obtenu lorsque l'état de mélange parfait est atteint, s'écarte de moins de 2 % de cette valeur finale de la lacune de miscibilité.

3. Procédé selon la revendication 1, **caractérisé en ce que** ledit au moins un dispositif de mélange consiste en un dispositif de mélange à buses, choisi dans le groupe constitué par des buses de mélange à courants croisés, à chambre, à éjecteur, coaxiales, des mélangeurs en Y et en T.

4. Procédé selon l'une quelconque des revendications précédentes, **caractérisé en ce que** le temps de mélange dans le dispositif de mélange est de 0,0001 s à 2 s.

5. Procédé selon l'une quelconque des revendications précédentes, **caractérisé en ce qu'**on fait passer le courant d'urée et le courant de prémélange à une vitesse comprise entre 5 et 100 m/s dans le dispositif de mélange.

6. Procédé selon l'une quelconque des revendications précédentes, **caractérisé en ce que** l'amine est choisie dans le groupe constitué par la butanediamine-1,4, la 2-éthylbutanediamine-1,4, l'octanediamine-1,8, la décanediamine-1,10, la dodécanediamine-1,12, la cyclohexanediamine-1,4, la 2-méthyl-, 4-méthyl-cyclohexanediamine-1,3, le 1,3- et le 1,4-diaminométhylcyclohexane, la 2-méthylpentanediamine-1,5, la 2,2,4- ou 2,4,4-triméthylhexanediamine-1,6, l'hexanediamine-1,6, la 3-aminométhyl-3,5,5-triméthylcyclohexylamine et des mélanges d'isomères 1,3- et 1,4-diaminométhylcyclohexane-3 (ou 4), 8 (ou 9)-bis(aminométhyl)-tricyclo[5.2.1.0^{2.6}]décane.

7. Procédé selon l'une quelconque des revendications précédentes, **caractérisé en ce que** l'alcool est choisi dans le groupe constitué par le méthanol, l'éthanol, le n-propanol, le n-butanol, l'isobutanol, le n-pentanol, l'isopentanol, le n-hexanol, les isohexanols, le 2-éthylhexanol, le décanol ou des mélanges des alcools nommés.
